# EUROPEAN PATENT APPLICATION

(11) **EP 1 405 909 A1**
(43) Date of publication of application: **07.04.2004**
(21) Application number: 02738769.5
(22) Date of filing: 19.06.2002
(51) Int. Cl.: C12N 15/09, C12Q 1/68

(54) **METHOD OF UNIFORMIZING DNA FRAGMENT CONTENTS AND SUBTRACTION METHOD**

(30) Priority: 19.06.2001 JP 2001184757
(71) Applicant: Eisai Co. Ltd, Tokyo 112-8088 (JP)
(72) Inventor: ONO, Yuichi, Ibaraki-shi, Osaka 567-0041 (JP); IMAI, Toshio, Kamigyo-ku, Kyoto-shi, Kyoto 602-8482 (JP)
(74) Representative: Bannerman, David Gardner
(86) International application number: PCT/JP2002/006109
(87) International publication number: WO 2002/103007

(57) **Abstract**

A method of normalizing contents of DNA fragments in a sample among respective DNA fragments, comprising: preparing DNA fragments in which adaptors each comprising an oligodeoxyribonucleotide are attached to both ends of each DNA fragment in the sample to form restriction enzyme recognition sites that do not exist in the DNA fragments in the sample, and at least a part between the restriction enzyme recognition sites at both ends, including the restriction enzyme recognition sites, is double-stranded; denaturing the prepared double-stranded DNA fragments; hybridizing the denatured DNA fragments under a condition that a part of the DNA fragments remains single-stranded; cleaving the hybridized double-stranded DNA fragments with a restriction enzyme having a cleavage site exclusively in the adaptors; and performing PCR using the obtained DNA fragments as templates and using a primer having a nucleotide sequence complementary to a nucleotide sequence of the adaptors before the cleavage; and a subtraction method comprising performing normalization by the method.

## Description

### Technical Field

The present invention relates to a method of normalizing contents of DNAs in a sample among respective DNAs, a method of detecting a difference in gene expression among cells, a method of producing a library of genes that are different in expression among cells, and a method of producing a probe specific to a gene that is different in expression among cells.

### Background Art

In order to understand life processes such as differentiation and development of cells, a pathologic condition such as tumorgenesis of cells, a response to a drug, and the like, investigation of a gene expression variation has been used often as one approach. As a method of detecting the difference in gene expression among the cells, a subtraction method, a differential display method, and the like are known.

Regarding the subtraction method, a subtraction method using a PCR (representational difference analysis (RDA) method), which has a higher sensitivity and a lower background compared with those of a conventional subtraction method, has been developed, and is being spread. According to this method, subtractive hybridization is repeated a plurality of times, whereby a very low background is realized, and is considered to be useful for subtraction among cell groups having a relatively small difference in gene expression. However, as a problem, the following is being understood: genes expressed in a large amount are concentrated more, so that genes expressed in a small amount cannot be isolated. As a method of overcoming the problem, a suppression subtractive hybridization (SSH) method has been developed. According to this method, subtraction is performed while the difference in an expression amount of genes is being normalized to some degree, and it is considered that this method also enables isolation of genes expressed in a small amount. As a normalization method, a method of performing partial hybridization between tester with an adaptor attached and drivers without an adaptor attached, and amplifying DNAs remaining single-stranded by a suppression PCR is used.

### Disclosure of the Invention

An object of the present invention is to provide a novel normalization method enabling a gene to be isolated based on a difference in an expression amount, even if the gene is expressed in a small amount. Another object of the present invention is to provide a method of detecting the difference in a gene expression among cells, including performing normalization using the above method, and a method of producing a library of genes that are different in an expression among cells by this detection method.

The inventors of the present invention have considered that a gene can be isolated based on the difference in an expression amount even if the gene is expressed in a small amount, provided that a normalization step can be added to an RDA method. As a result of the study, the inventors have found a normalization method that can be used for normalizing contents of DNAs in a reaction liquid among respective DNAs in an RDA method. Thus, the inventors have achieved the present invention based on this finding.

The present invention provides the following.
(1) A method of normalizing contents of DNA fragments in a sample among respective DNA fragments, comprising:
   preparing DNA fragments in which adaptors each comprising an oligodeoxyribonucleotide are attached to both ends of each DNA fragment in the sample to form restriction enzyme recognition sites that do not exist in the DNA fragments in the sample, and at least a part between the restriction enzyme recognition sites at both ends, including the restriction enzyme recognition sites, is double-stranded;
   denaturing the prepared double-stranded DNA fragments;
   hybridizing the denatured DNA fragments under a condition that a part of the DNA fragments remains single-stranded;
   cleaving the hybridized double-stranded DNA fragments with a restriction enzyme having a cleavage site exclusively in the adaptors; and
   performing PCR using the obtained DNA fragments as templates and using a primer having a nucleotide sequence complementary to a nucleotide sequence of the adaptors before the cleavage.
(2) A method of detecting a difference in a gene expression among cells, which comprises performing subtractive hybridization at least once, and further comprising performing normalization by the method as defined in the item (1) before or after performing at least one of the subtractive hybridization(s).
(3) The method according to the item (2), in which at least one of the subtractive hybridization(s) generates double-stranded DNA fragments with adaptors attached to both ends, and the normalization is performed after the subtractive hybridization.
(4) The method according to the item (3), in which the subtractive hybridization is performed at least twice, and the first subtractive hybridization generates the double-stranded DNA fragments with the adaptors attached to both the ends.
(5) A method of producing a library of genes which are different in an expression among cells, comprising cloning genes which are detected for a difference in the expression by the method as defined in any one of the items (2) to (4).
(6) A method of producing a probe specific to a gene which is different in an expression among cells, comprising preparing a fragment of a gene which is detected for a difference in the expression by the method as defined in any one of the items (2) to (4).
(7) A kit for use in the method as defined in the item (1), comprising an adaptor comprising an oligodeoxyribonucleotide to be attached to both ends of DNA fragments in a sample, a restriction enzyme having a cleavage site in the adaptor, and a primer for PCR having a nucleotide sequence complementary to a nucleotide sequence of the adaptor before cleavage with the restriction enzyme.

### Brief Description of the Drawings

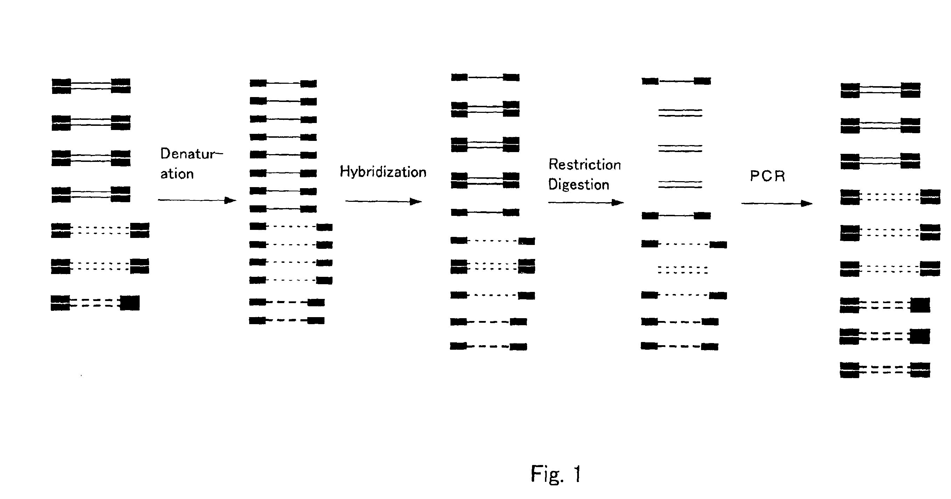

FIG. 1 is a diagram illustrating the principle of normalization of the present invention.
FIG. 2 is an electrophoresis photograph showing a result of precision verification of an N-RDA method.
FIG. 3 is an electrophoresis photograph showing the result of precision verification of an N-RDA method.
FIG. 4 is an electrophoresis photograph showing the result of precision verification of an N-RDA method with VE: umbilical vein endothelial cell, AE: umbilical artery endothelial cell, ADM: dermal microvascular endothelial cell, TE: tonsil endothelial cell, BME: brain microcapillary endothelial cell, MS1: mouse blood vessel endothelial cell line, and Eph4: mouse epithelial cell line.
FIG. 5 is an electrophoresis photograph showing the result of an expression in various kinds of blood vessel endothelial cells, of clones isolated by an N-RDA method.

### Best Mode for carrying out the Invention

Hereinafter, the present invention will be described in detail.

The present invention relates to a method of normalizing contents of DNA fragments in a sample among respective DNA fragments (hereinafter, which may also be referred to as a "normalization method"), characterized by including:
(a) preparing DNA fragments in which adaptors each comprising an oligodeoxyribonucleotide are attached to both ends of each DNA fragment in the sample to form restriction enzyme recognition sites that do not exist in the DNA fragments, and at least a part between the restriction enzyme recognition sites at both ends, including the restriction enzyme recognition sites, is made double-stranded;
(b) denaturing the prepared double-stranded DNA fragments;
(c) hybridizing the denatured DNA fragments under a condition that a part of the DNA fragments remains single-stranded;
(d) cleaving the hybridized double-stranded DNA fragments by a restriction enzyme having a cleavage site exclusively in the adaptors; and
(e) performing PCR using the obtained DNA fragments as templates and using a primer having a nucleotide sequence complementary to a nucleotide sequence of the adaptors before the cleavage.

There is no limit to a sample as long as it contains DNA fragments. Examples of the DNA fragments include a cDNA synthesized from mRNA prepared from cells or fragments of the cDNA, DNA fragments of genes that are different in an expression among cells, obtained by subtractive hybridization.

The principle of normalization according to the present invention will be described with reference to FIG.
1. When double-stranded cDNAs are denatured, and hybridized (annealed) again, cDNAs become double-stranded in the decreasing order of an amount, and cDNAs in a small amount are unlikely to be double-stranded. Therefore, if only the cDNAs remaining single-stranded are amplified at a time when a reaction proceeds to some degree, the difference in an existing amount can be adjusted. The present invention is mainly characterized in that decomposition with a restriction enzyme is adopted so as to amplify only the cDNAs remaining single-stranded. Hereinafter, each process will be described.

In the step (a), DNA fragments are prepared in which adaptors each comprising an oligodeoxyribonucleotide are attached to both ends of each DNA fragment in a sample to form restriction enzyme recognition sites that do not exist in the DNA fragments in the sample, and at least a part between the restriction enzyme recognition sites, including the restriction enzyme recognition sites, is made double-stranded.

The nucleotide sequence of the adaptor is made to be cleaved only in the adaptor by a restriction enzyme used in the step (4), considering the nucleotide sequences of the DNA fragments. Because of this, the restriction enzyme used in the step (d) has a cleavage site only in the adaptor. The length of the adaptor is generally 20 to 30 bases.

Formation of restriction enzyme recognition sites means that the DNA fragment with adaptors attached thereto has restriction enzyme recognition sites, and includes both the presence of the restriction enzyme recognition sites in the adaptors and the formation of the restriction enzyme recognition sites due to the binding between the adaptors and the DNA fragment. In the case where the DNA fragments in a sample have a common nucleotide sequence at both ends, it is easy to design the nucleotide sequences of the adaptors in such a manner that restriction enzyme recognition sites are formed due to the binding between the adaptors and the DNA fragments.

A part to be double-stranded may be at least a part between the restriction enzyme recognition sites at both ends inclusive of the restriction enzyme recognition sites, or may equal the full length.

In the case where the DNA fragments are single-stranded, they are made to be double-stranded by conventional methods in the field of genetic engineering. The adaptors may be attached to the DNA fragments before or after the DNA fragments are made to be double-stranded. More specifically, after the adaptors are attached to the single-stranded DNA fragments, required parts may be made to be double-stranded. Alternatively, the DNA fragments and the adaptors are made to be double-stranded, separately, and then, they may be attached to each other. Alternatively, it is also possible that the DNA fragments are made to be double-stranded, the adaptors are attached to one strand, and a single-stranded part is filled in to make a required part double-stranded. In the case where the DNA fragments are double-stranded, the above-mentioned latter two methods may be mentioned as a method of attaching adaptors. The attachment can be performed by a conventional method in the field of genetic engineering.

Furthermore, in the case where the DNA fragments are double-stranded having nucleotide sequences corresponding to the adaptors at both ends, they may be used as they are.

In the step (b), the double-stranded DNA fragments prepared in the step (a) are denatured. The denaturation can be performed by a conventional method in the field of genetic engineering. For example, denaturation by heating is mentioned. In this case, the condition is varied depending upon the nucleotide sequences of the double-stranded DNA fragments. For example, denaturation is performed at 94°C to 100°C for 3 to 5 minutes.

In the step (c), the DNA fragments denatured in the step (b) are hybridized under a condition that a part of the DNA fragments remains single-stranded.

The hybridization of the denatured DNA fragments is also called annealing, which can be performed by a conventional method in the field of genetic engineering. In this process, DNA fragments existing in a large amount are hybridized quickly, and the DNA fragments existing in a small amount are hybridized slowly. Consequently, the difference in an existing amount of the DNA fragment remaining single-stranded is decreased. Thus, by hybridizing the DNA fragments under a condition that a part of the DNA fragments remains single-stranded, the contents of single-strands of the respective DNA fragments are normalized. As is understood from this, "normalization" in the present invention includes decreasing the difference in the DNA fragment contents.

There is no particular limit to the condition for allowing a part of the DNA fragments to remain single-stranded, as long as the treatment of the subsequent process can be started before double-strands are formed completely. For example, the following may be performed: hybridization is started by decreasing a temperature, and hybridization is completed before double-strands are formed completely. The specific condition in this case is varied depending upon the nucleotide sequences of the DNA fragments. For example, the hybridization is performed at 65°C to 70°C for 12 to 20 hours. Whether or not a part of the DNA fragments remains single-stranded can be confirmed by gel electrophoresis, and it would be easy for those skilled in the art to select an appropriate condition. It is considered that, by adjusting the concentration of the DNA fragments and the time of hybridization, only the DNA fragments existing in a relatively large amount can be decreased, or the DNA fragments existing in a very small amount can be increased.

In the step (d), the double-stranded DNA fragments hybridized in the step (c) are cleaved by a restriction enzyme having a cleavage site only in the adaptors.

The restriction enzyme is appropriately selected based on the sequences of the adaptors and the DNA fragments in the sample. The adaptors also include the attachment sites of the adaptors to the DNA fragments.

The cleavage condition is varied depending upon the restriction enzyme. For example, the cleavage is performed at 30 to 37°C and pH 7.5 to 8.5 for 30 to 120 minutes.

In the step (e), PCR is performed using the DNA fragments obtained in the step (d) as templates, and using a primer having a nucleotide sequence complementary to the nucleotide sequence of the adaptors before cleavage.

The PCR can be performed by a conventional method. It would be easy for those skilled in the art to set an appropriate condition, considering the nucleotide sequences of the templates and the primer. The length of the primer is generally 20 to 30 bases. An oligonucleotide used as the adaptor may be used as the primer.

The present invention also provides a kit for use in the normalization method of the present invention. This kit comprises an adaptor comprising an oligodeoxyribonucleotide to be attached to both ends of the DNA fragments in a sample, a restriction enzyme having a cleavage site in the adaptor, and a primer for PCR having a nucleotide sequence complementary to the nucleotide sequence of the adaptor before cleavage with the restriction enzyme.

The adaptor, the restriction enzyme, and the primer for PCR are as described with reference to the above-mentioned normalization method of the present invention.

The kit of the present invention may include a part of or an entirety of reagents (buffer, etc.) required for cleavage with a restriction enzyme and reagents (buffer, dNTP, DNA polymerase, etc.) required for PCR, in addition to the adaptor, the restriction enzyme, and the primer for PCR.

According to the subtractive hybridization, mRNAs or cDNAs prepared from two different kinds of cells are hybridized, and cDNAs that are not hybridized are isolated, whereby specific cDNAs (i.e., that are different in an expression amount among cells) are detected. The important point of influencing the efficiency of this method is the efficiency of hybridization. Genes expressed in a small amount have a low hybridization efficiency, compared with those expressed in a large amount. Therefore, it is difficult to isolate the genes expressed in a small amount. Thus, by normalizing the contents of the DNA fragments in a sample by combination of the normalization method of the present invention with the subtractive hybridization, the difference in a gene expression among cells can be detected even in genes expressed in a small amount.

Thus, the present invention also provides a method of detecting the difference in a gene expression among cells, comprising performing subtractive hybridization at least once (hereinafter, which may also be referred to as a "detection method"), in which normalization is performed by the normalization method of the present invention before or after performing at least one of the subtractive hybridization(s).

The detection method of the present invention may be the same as that of detecting the difference in a gene expression among cells, comprising performing conventional subtractive hybridization at least once, except that normalization is performed by the normalization method of the present invention before or after performing at least one of the subtractive hybridization(s).

In the case where at least one of the subtractive hybridization(s) generates double-stranded DNA fragments with adaptors attached to both ends, the above-mentioned step (a) of the normalization method of the present invention can be simplified. Therefore, it is preferable to perform normalization after the subtractive hybridization.

It is known that even a small difference in an expression amount can be detected by performing subtractive hybridization twice or more. As a method of performing subtractive hybridization twice or more, a representational difference analysis (RDA) method is known.

According to the RDA method, the following subtractive hybridization is performed a plurality of times. That is, cDNA fragments (tester) with an oligonucleotide called adaptor attached and cDNA fragments (driver) without adaptor attached are denatured and then hybridized. Then, among the hybridized double-strands, strands complementary to those in which the adaptor portions are single-stranded are synthesized to be double-stranded over the full length, and thereafter, PCR is performed using a primer complementary to the adaptors. By adding the cDNA fragments without the adaptors attached in an excess amount as driver, only those which are specific to the tester form double-strands with the adaptors at both ends, and only such cDNA fragments are amplified by the PCR. For example, in the case of using the driver in a 100-fold amount of the tester, non-specific genes are decreased to 1/100, and thus, specific genes are concentrated 100-fold. However, in the case where the difference in an existing amount between the tester and the driver is very small, it is difficult to isolate specific genes with about 100-fold concentration. Thus, a subtraction operation is repeated twice or more (generally, 3 to 4 times) to completely remove non-specific genes, whereby genes that are specifically expressed among cells with a smaller difference can be isolated.

However, genes expressed in a small amount have a lower efficiency of hybridization, compared with those expressed in a large amount. Therefore, the difference in an amount is increased as subtractive hybridization is repeated. According to the general RDA method, for example, the ratio of the driver is considerably increased to 100-fold (first time), 800-fold (second time), and 400000-fold (third time). This is considered to be effective for decreasing a background. However, there is a limit to the amount of cDNAs that can be used for the driver. Actually, the ratio is increased by decreasing the amount of the tester. Therefore, the frequency of forming double-strands of the genes expressed in a small amount is decreased more, and only genes expressed in a large amount are amplified preferentially.

Thus, in a method in which subtractive hybridization is performed twice or more, such as the RDA method, it is preferable to perform normalization by the normalization method of the present invention before or after performing at least one of the subtractive hybridization(s). Furthermore, according to the subtractive hybridization in the RDA method, double-stranded DNA fragments with adaptors attached to both ends are generated. Therefore, if the adaptors in the RDA method can be used as the adaptors in the normalization method of the present invention, it is preferable that normalization is performed after the hybridization.

Regarding the subtractive hybridization and the normalization, it is preferable that the normalization is performed after the first subtraction, and then, the second and subsequent subtractions are performed. In particular, it is considered that, by performing normalization under a condition that only genes in a relatively large amount are decreased, genes expressed in a relatively small amount can be isolated easily.

In the RDA method combined with the normalization method of the present invention, as described above, it is preferable that the ratio of the tester to the driver is decreased to increase the concentration of the tester so as to suppress the increase in the difference in an existing amount as the subtraction is performed. For example, 30 to 50-fold, 100 to 300-fold, and 1000 to 5000-fold drivers are used in the first, second, and third subtractive hybridizations, respectively. The step of normalization for adjusting the difference in an expression amount is added between the first and second subtractive hybridizations.

According to the RDA method, specific cDNA fragments first cloned are added to the driver, whereby other kinds of cDNA fragments can be amplified, and much more kinds of genes can be isolated efficiently.

As a method of performing subtraction while normalizing the difference in an expression amount of genes to some degree, a suppression subtractive hybridization (SSH) method is known. However, according to the SSH method, subtractive hybridization is performed only once. Therefore, it is impossible to sufficiently remove a background, and it may not be expected that the genes are concentrated to such a degree that specific cDNAs can be seen as a band, depending upon the degree of an expression amount. According to the RDA method combined with the normalization method of the present invention, subtractive hybridization is performed a plurality of times. Therefore, even genes expressed in a much smaller amount are considered to be isolated.

The cells for detecting the difference in a gene expression by the detection method of the present invention are not particularly limited. The detection method of the present invention can be applied to the detection of the difference in a gene expression among cells derived from different tissues and among the same cells cultured under different conditions.

Furthermore, the present invention provides a method of producing a library of genes which are different in an expression among cells, comprising cloning genes whose difference in an expression is detected by the detection method of the present invention, and a method of producing a probe specific to a gene which is different in an expression among cells, comprising preparing a fragment of the gene whose difference in an expression is detected by the detection method of the present invention.

The production of the library by cloning of the genes and the production of the probe by the preparation of the gene fragment can be performed by a conventional method, except that the gene or genes are detected for the difference in an expression by the detection method of the present invention.

### Examples

Hereinafter, the present invention will be described more specifically by way of examples.

### Example 1

### [Protocol]

### (1) Preparation of adaptor

Oligonucleotides (ad2S and ad2A) having nucleotide sequences of SEQ ID No: 1 and SEQ ID No: 2 are annealed to prepare an adaptor. The annealing is performed under the following condition. TE represents 10 mM Tris/HCl (pH 8.0) buffer containing 1 mM EDTA.

### Reaction mixture composition

| | |
|---|---|
| 0.2 M NaCl | 2 µl |
| 500 µM ad2S (in TE) | 9 µl |
| 500 µM ad2A (in TE) | 9 µl |

### Reaction temperature and time

94°C, 5 minutes → 50°C, 5 minutes → 47°C, 5 minutes → 44°C, 5 minutes → 41°C, 5 minutes → 38°C, 5 minutes → 35°C, 5 minutes → 32°C, 5 minutes → 29°C, 5 minutes → 4°C

After annealing, 30 µl TE is added to obtain 100 µM of an adaptor solution (ad2).

Similarly, using oligonucleotides (ad3S and ad3A) having nucleotide sequences of SEQ ID No: 3 and SEQ ID No: 4, oligonucleotides (ad4S and ad4A) having nucleotide sequences of SEQ ID No: 5 and SEQ ID No: 6, oligonucleotides (ad5S and ad5A) having nucleotide sequences of SEQ ID No: 7 and SEQ ID No: 8, oligonucleotides (ad13S and ad13A) having nucleotide sequences of SEQ ID No: 9 and SEQ ID No: 10, and oligonucleotides (ad14S and ad14A) having nucleotide sequences of SEQ ID No: 11 and SEQ ID No: 12, adaptor solutions (ad3, ad4, ad5, ad13, and ad14, respectively) are prepared.

### (2) cDNA synthesis

Double-stranded cDNAs are synthesized from a sample, using a TAKARA cDNA synthesis kit. The thus obtained cDNAs are purified, using a QIAquick PCR purification kit (hereinafter, referred to as "QIAquick") (eluted with 20 µl of TE). The purified cDNAs are *Rsa*I-digested (TAKARA *Afa*I) in a 100-µl system, and purified with QIAquick. In the case of using 1 µg of poly(A)+ RNA, the cDNAs are eluted with 20 µl of TE. In the case of using 1 µg of total RNAs, the cDNAs are eluted with 50 µl of TE, precipitated with ethanol, and dissolved in 2 µl of TE.

### (3) Binding of cDNA to adaptor ad2

The cDNAs are bound to the adaptor ad2, using the TAKARA DNA ligation kit Ver 2. 1 µl of cDNAs, 1 µl of 100 µM ad2, 2 µl of solution II, and 4 µl of solution I are mixed, and allowed to react at 16°C for 2 hours, whereby a cDNA solution (ad2 lig cDNA) with the adaptors ad2 added thereto is obtained.

### (4) Amplification of cDNA with adaptor ad2 attached thereto

A PCR is performed under the following condition:

### Reaction mixture composition

| | |
|---|---|
| 10 × ExTaq | 5 µl |
| 2.5 mM dNTP | 4 µl |
| ExTaq | 0.25 µl |
| 100 µM ad2S | 0.5 µl |
| ad2 lig cDNA | 1 µl or 0.1 µl |
| DW | 39.25 µl |

### Reaction temperature and time

72°C, 5 minutes → (94°C, 30 seconds → 65°C, 30 seconds → 72°C, 2 minutes) × 15 cycles → 72°C, 2 minutes

When 10 µl of the reaction liquid is electrophoresed, if products are maximally observed (about 500 ng/µl) at 1 µl of the templates, and observed in some amounts even at 0.1 µl, it is considered that 10⁷ or more kinds are contained.

The remaining portion of 40 µl at 1 µl of template is purified with QIAquick (eluted with 40 µl of TE), whereby an amplified cDNA solution (cDNA ad2 amp) is obtained.

### (5) Production of tester and driver

Most of cDNAs contained in cDNA ad2 amp are single-stranded; therefore, a PCR is performed again under the following condition:

### Reaction mixture composition

| | |
|---|---|
| 10 × ExTaq | 5 µl |
| 2.5 mM dNTP | 4 µl |
| ExTaq | 0.25 µl |
| 100 µM ad2S | 0.5 µl |
| cDNA ad2 amp | 0.5 µl |
| DW | 39.75 µl |

### Reaction temperature and time

94°C, 2 minutes → (94°C, 30 seconds → 65°C, 30 seconds → 72°C, 2 minutes) × 5 cycles → 72°C, 2 minutes

One PCR run for tester and 5 PCR runs per subtraction cycle for driver (20 PCR runs in total) are performed. Then, 10 µl of the reaction mixture is electrophoresed and checked (about 100 ng/10 µl), and the rest is purified with QIAquick (columns: one column for tester and four columns for driver) (eluted with 20 µl of TE), whereby an amplified cDNA solution (cDNA ad2 amp (5 cycles)) is obtained. This solution is *Rsa*I-digested under the following condition:

### Reaction mixture composition

| | |
|---|---|
| cDNA ad2 amp (5 cycles) | 20 µl |
| 10 × T | 10 µl |
| BSA | 10 µl |
| *Rsa*I (10 u/µl) | 2 µl |
| DW | 58 µl |
| (One for tester, four for driver) | |

The products for driver are reacted at 37°C for 2 hours, and a restriction enzyme is inactivated by a treatment at 70°C for 5 minutes, whereby a complete driver solution (cDNA ad2 amp RsaI) is obtained.

The products for tester are reacted at 37°C for one hour, and purified with QIAquick (eluted with 20 µl of TE) to obtain a digested product (cDNA ad2 amp RsaI).

The digested product is bound to the adaptor ad3, using the TAKARA DNA ligation kit Ver2. Then, 3 µl of cDNA ad2 amp RsaI, 1 µl of 100 µM ad3, 4 µl of solution II, and 8 µl of solution I are mixed and allowed to react at 16°C for one hour, and a restriction enzyme is inactivated by treatment at 70°C for 5 minutes, whereby a complete tester solution (cDNA ad3 lig) is obtained.

### (6) First subtraction

16 µl of the tester solution (cDNA ad3 lig) and 100 µl of the driver solution (cDNA ad2 amp RsaI) are mixed (T:D = 1:40), and the mixture is purified with QIAquick (eluted with 50 µl of TE) and precipitated with ethanol.

Then, 50 µl of cDNAs (tester & driver mixture), 5 µl of 3 M sodium acetate, 1 µl of 10 mg/ml of glycogen, and 125 µl of ethanol are mixed, and centrifuged immediately for 5 minutes. The resultant precipitate is rinsed with 70 % ethanol and dried. Thereafter, the precipitate is dissolved in 1 µl of 1 × PCR buffer. The solution is transferred to a PCR tube, and overlaid with mineral oil. After the solution is retained at 98°C for 5 minutes, 1 µl of 1 x PCR buffer + 1 M NaCl is added thereto and mixed together, and treatment (98°C, 5 minutes → 68°C, 16 hours) is performed.

Then, 10 µl of TE is added to the solution and collected in a new tube. Thereafter, a PCR is effected under the following condition:

### Reaction mixture composition

| | |
|---|---|
| 10 × Taq | 5 µl |
| 2.5 mM dNTP | 4 µl |
| Taq | 0.5 µl |
| 100 µM ad3S | 0.5 µl |
| Treated sample | 2 µl |
| DW | 38 µl |

### Reaction temperature and time

72°C, 5 minutes → (94°C, 30 seconds → 65°C, 30 seconds → 72°C, 2 minutes) × 10 cycles → 4°C

The solution in an amount corresponding to 5 runs is purified in one column with QIAquick and precipitated with ethanol. The precipitate is rinsed with 70% ethanol, and dried. Then, 5 µl of TE, 5 µl of 10 × mung bean buffer, 38.5 µl of DW, and 1.5 µl of mung bean nuclease are added to the solution, and the mixture is allowed left at room temperature for 20 minutes.

After 5 µl of 0.05 M EDTA and 45 µl of 50 mM Tris 7.6/100 mM NaCl are added, the mixture is purified with QIAquick (eluted with 30 µl of TE) to obtain a purified product (S1 pure).

A PCR is performed under the following condition:

### Reaction mixture composition

| | |
|---|---|
| 10 × Taq | 5 µl |
| 2.5 mM dNTP | 4 µl |
| Taq | 0.25 µl |
| 100 µM ad3S | 0.5 µl |
| S1 pure | 2 µl |
| DW | 38.25 µl |

### Reaction temperature and time

94°C, 2 minutes → (94°C, 30 seconds → 65°C, 30 seconds → 72°C, 2 minutes) × 12 cycles → 72°C, 2 minutes

10 µl of the reaction liquid is electrophoresed to confirm that amplification is completed. The rest of the reaction mixture is purified with QIAquick (eluted with 40 µl of TE) to obtain an amplified product (S1 amp). In the case where it is desired to confirm the pattern of a band, the second PCR is also performed under the conditions of 5 and 7 cycles, and electrophoresis is performed.

### (7) Normalization

1 µl of 2 × PCR buffer is added to 1 µl of S1 amp diluted 5-fold with TE, and mineral oil is overlaid. After the resultant solution is allowed to stand at 98°C for 5 minutes, 2 µl of 1 × PCR buffer + 1 M NaCl is added and mixed together, and treatment (98°C, 5 minutes → 68°C, 16 hours) is performed.

After 10 µl of 10 × T buffer, 10 µl of BSA, 3 µl of *Rsa*I, and 73 µl of DW are added to the resultant solution, and the solution is treated at 37°C for one hour, the solution is purified with QIAquick (eluted with 20 µl of TE) to obtain a purified product (N1 pure).

A PCR is performed under the following condition:

### Reaction mixture composition

| | |
|---|---|
| 10 × ExTaq | 5 µl |
| 2.5 mM dNTP | 4 µl |
| ExTaq | 0.25 µl |
| 100 µM ad3S | 0.5 µl |
| N1 pure | 2 µl |
| DW | 38.25 µl |

### Reaction temperature and time

94°C, 2 minutes → (94°C, 30 seconds → 65°C, 30 seconds → 72°C, 2 minutes) × 11 to 15 cycles → 72°C, 2 minutes

Then, the rest of the sample after a certain number of cycles where about 50 ng is contained (to such a degree as to be slightly observed) when 10 µl of the reaction liquid is electrophoresed, is purified with QIAquick (eluted with 20 µl of TE), and digested with *Rsa*I (37°C for one hour). This is purified with QIAquick (eluted with 20 µl of TE) to obtain a digested product (N1 amp RsaI).

The digested product is bound to the adaptor ad4, using the TAKARA DNA ligation kit Ver2. Then, 2 µl of N1 amp RsaI, 1 µl of 100 µM ad4, 3 µl of solution II, and 6 µl of solution I are mixed and allowed to react at 16°C for one hour, and a restriction enzyme is inactivated by treatment at 70°C for 5 minutes, whereby a tester solution for the second time (N1 amp ad4 lig) is obtained.

### (8) Second subtraction

12 µl of the tester solution (N1 amp ad4 lig) and 100 µl of the driver solution (cDNA ad2 amp RsaI) are mixed (T:D = 1:100), and the mixture is purified with QIAquick (eluted with 50 µl of TE) and precipitated with ethanol.

Thereinafter, the process is performed in the same manner as in the first subtraction (3 to 7 cycles for PCR after treatment with mung bean nuclease).

Then, the rest of the sample after a certain number of cycles where 50 ng is contained (to such a degree as to be slightly observed) when 10 µl of the reaction liquid is electrophoresed, is purified with QIAquick (eluted with 20 µl of TE), and digested with *Rsa*I (37°C for one hour). This is purified with QIAquick (eluted with 20 µl of TE) to obtain a digested product (NS2 amp RsaI).

The digested product is bound to the adaptor ad5, using the TAKARA DNA ligation kit Ver2. Then, 1 µl of NS2 amp RsaI diluted 1/5-fold, 1 µl of 100 µM ad5, 2 µl of solution II, and 4 µl of solution I are mixed and allowed to react at 16°C for one hour, and a restriction enzyme is inactivated by treatment at 70°C for 5 minutes, whereby a tester solution for the third time (NS2 amp ad5 lig) is obtained.

### (9) Third subtraction

8 µl of the tester solution (NS2 amp ad5 lig) and 100 µl of the driver solution (cDNA ad2 amp RsaI) are mixed (T:D = 1:1000), and the mixture is purified with QIAquick (eluted with 50 µl of TE) and precipitated with ethanol.

Thereinafter, the process is performed in the same way as in the first subtraction (PCR is performed in 6, 8, and 12 cycles after treatment with mung bean nuclease).

At this time, if the number of kinds of amplified products is small, it is considered that products are double-stranded. Therefore, the products are cloned to a TA vector or the like as they are. If the number of kinds is large, the genes are diluted 100-fold, and 5 cycle-PCR is performed to make the products into double-strands. Thereafter, cloning is performed.

### Example 2

### [Precision Verification 1 of an experiment system (model system)]

In order to check which degree of an expression amount and of a difference in an expression amount genes can be isolated to by the method described in Example 1, it was checked whether or not φX174 fragments can be isolated, using, as tester and driver, cDNAs prepared from a blood vessel endothelial cell line MS1 to which DNA *Rsa*I digestion fragments derived from bacteriophage φX174 are added.

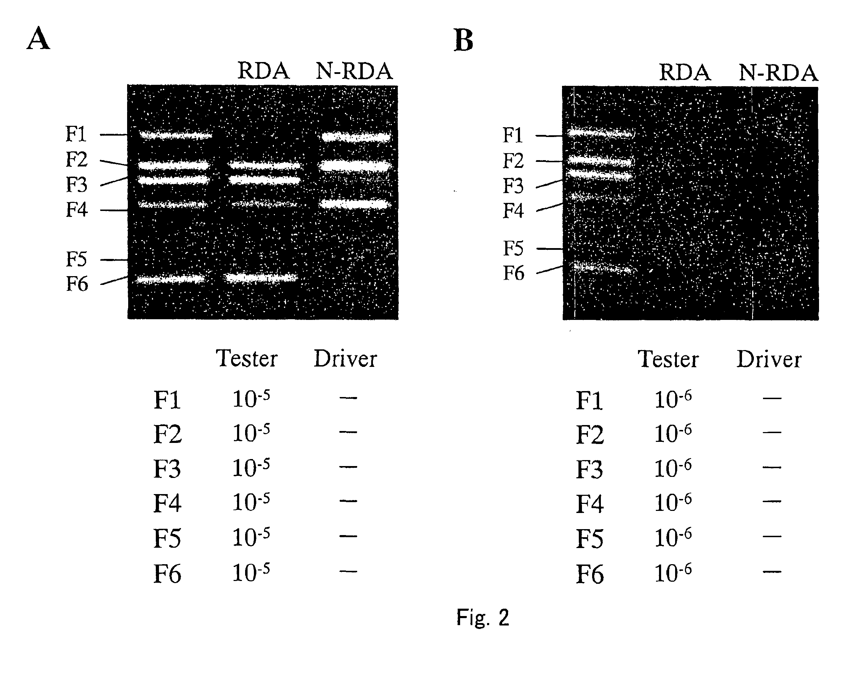

FIG. 2, A shows electrophoresis of final PCR products obtained in the case where the method described in Example 1 (N-RDA method) and the method omitting the process of normalization (RDA method) were performed, using MS1 cDNAs to which 6 kinds of φX174 *Rsa*I fragments were added in a ratio of 1/10⁵ as tester and using MS1 cDNAs as driver. Lane 1 shows the result of the electrophoresis of 6 kinds of added φX174 *Rsa*I fragments (F1 to F6), Lane 2 shows the result of the electrophoresis of the amplified product after the third subtraction of the RDA method, and Lane 3 shows the result of the electrophoresis of the amplified product after the third subtraction of the N-RDA method. In any of the methods, 5 out of 6 kinds were amplified. This clarified that almost all the specific fragments existing in a ratio of 1/10⁵ can be isolated.

FIG. 2, B shows the result obtained in the case of adding φX174 *Rsa*I fragments in a ratio of 1/10⁶ in the same manner as in the A. In this ratio, φX174 *Rsa*I fragments were not amplified in any of the methods. Thus, it was clarified that the limit of these methods is between 1/10⁵ and 1/10⁶.

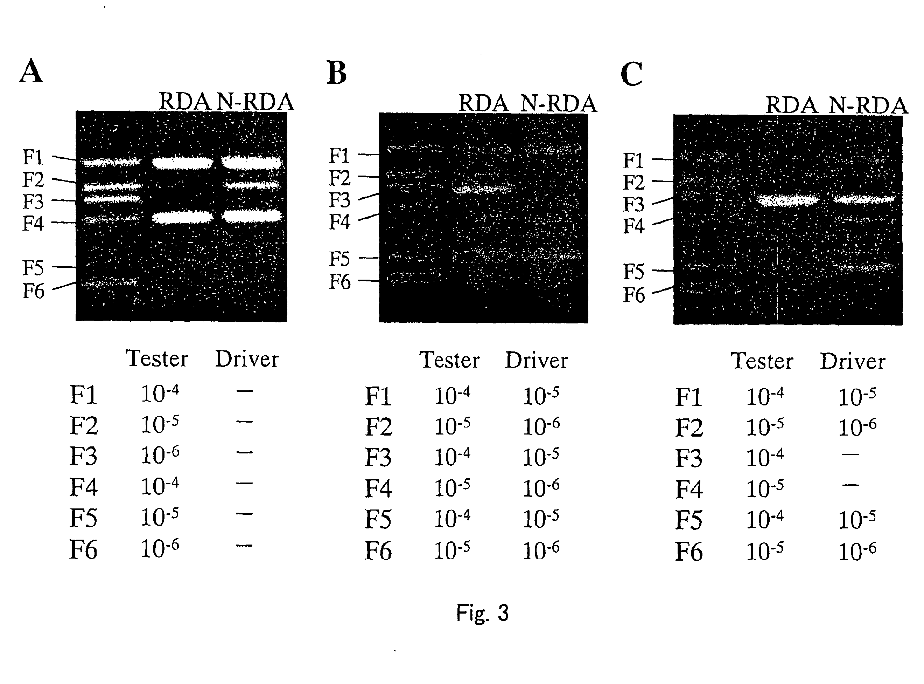

FIG. 3, A shows the result (result of electrophoresis of an amplified product after the third subtraction) obtained in the case of adding 6 kinds of φX174 *Rsa*I fragments in each ratio shown in the figure. According to the RDA method, F1 and F4 added in a ratio of 1/10⁴ were amplified efficiently, and F2 added in a ratio of 1/10⁵ was amplified as well. On the other hand, according to the N-RDA method, the amount of F2 was increased compared with the RDA method, and F5 was also amplified. Therefore, it was confirmed that the effect of normalization was exhibited.

Next, in order to study whether isolation can be performed even with a 10-fold expression difference, an experiment was performed by adding the same φX174 *Rsa*I fragments to the driver in a ratio of 1/10 of the tester (FIG. 3, B). According to the RDA method, it was confirmed that those contained in a ratio of 1/10⁴ in the tester (F1, F3 and F5) were amplified even with a 10-fold difference, but those existing in a ratio of 1/10⁵ were not amplified. In contrast, according to the N-RDA method, F2 and F4 contained in a ratio of 1/10⁵ were also amplified.

Furthermore, as a model closest to the practical case, the case has been studied in which φX174 *Rsa*I fragments that are different in an existing amount and in an existing amount ratio between tester and driver are added (FIG. 3, C). Consequently, according to the RDA method, it was confirmed that specific F3 in a large amount was amplified preferentially, and F1, F4, and F5 were barely amplified. In contrast, according to the N-RDA method, the amounts of F1, F4, and F5 were increased, and F2 was further amplified.

From the above-mentioned results, the effect of normalization was confirmed under any of the conditions. Thus, it is apparent that the N-RDA method is very effective for isolating as many genes as possible at once.

### Example 3

### [Precision Verification 2 of an experiment system (subtraction among blood vessel endothelial cells derived from various kinds of normal human tissues)]

Subtraction was performed among some blood vessel endothelial cells derived from normal human tissues. As materials, umbilical vein endothelial cell (VE), umbilical artery endothelial cell (AE), dermal microvascular endothelial cell (ADM), tonsil endothelial cell (TE), and brain microcapillary endothelial cell (BME) were used. Furthermore, as a comparison between cells with a relatively large difference, subtraction was performed between a mouse blood vessel endothelial cell line (MS1) and a mouse epithelial cell line (Eph4).

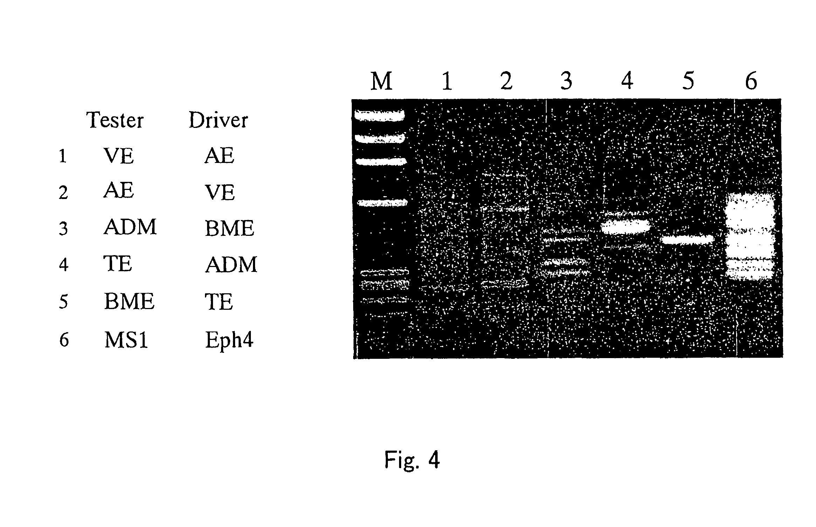

FIG. 4 shows combinations of subtraction. In the subtraction between blood vessel endothelial cells, several to several tens of bands were amplified. In contrast, in the subtraction of MS1/Eph4, a great number of bands were amplified. This result is considered to reflect the number of genes of specific expression.

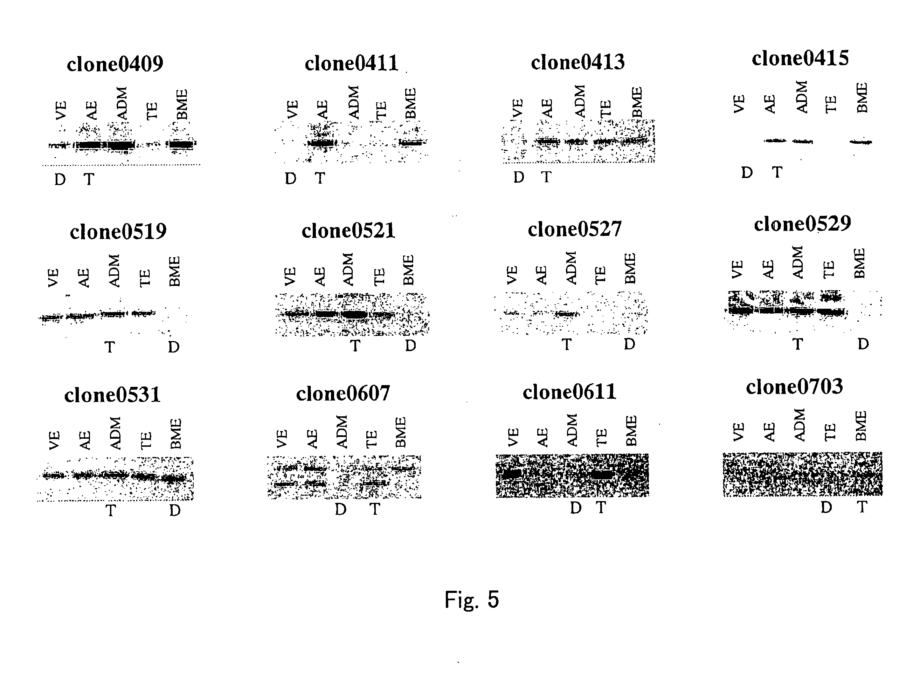

Next, in order to confirm whether or not the amplified genes actually exhibit specific expression, the bands amplified by the subtraction among blood vessel endothelial cells were cloned, and cDNAs prepared from the respective blood vessel endothelial cells were subjected to southern blotting (FIG. 5). Consequently, it was confirmed that 11 kinds out of 12 kinds were completely specific or had a 10-fold or more difference in an expression, and it was clarified that the remaining one kind also had a several-fold difference. From the above result, it was confirmed that, according to the N-RDA method, genes specific in an expression can be isolated with a low background even among cells actually having a relatively small difference.

### Industrial Applicability

According to the present invention, the DNA fragment contents in a sample can be normalized. By combining this normalization method with subtractive hybridization, even genes existing in a small expression amount can be isolated based on the difference in an expression amount.

## Claims

1. A method of normalizing contents of DNA fragments in a sample among respective DNA fragments, comprising:
preparing DNA fragments in which adaptors each comprising an oligodeoxyribonucleotide are attached to both ends of each DNA fragment in the sample to form restriction enzyme recognition sites that do not exist in the DNA fragments in the sample, and at least a part between the restriction enzyme recognition sites at both ends, including the restriction enzyme recognition sites, is double-stranded;
denaturing the prepared double-stranded DNA fragments;
hybridizing the denatured DNA fragments under a condition that a part of the DNA fragments remains single-stranded;
cleaving the hybridized double-stranded DNA fragments with a restriction enzyme having a cleavage site exclusively in the adaptors; and
performing PCR using the obtained DNA fragments as templates and using a primer having a nucleotide sequence complementary to a nucleotide sequence of the adaptors before the cleavage.

2. A method of detecting a difference in a gene expression among cells, which comprises performing subtractive hybridization at least once, and further comprising performing normalization by the method as defined in claim 1 before or after performing at least one of the subtractive hybridization(s).

3. A method according to claim 2, wherein at least one of the subtractive hybridization(s) generates double-stranded DNA fragments with adaptors attached to both ends, and the normalization is performed after the subtractive hybridization.

4. A method according to claim 3, wherein the subtractive hybridization is performed at least twice, and the first subtractive hybridization generates the double-stranded DNA fragments with the adaptors attached to both the ends.

5. A method of producing a library of genes which are different in an expression among cells, comprising cloning genes which are each detected for a difference in the expression by the method as defined in any one of claims 2 to 4.

6. A method of producing a probe specific to a gene which is different in an expression among cells, comprising preparing a fragment of a gene which is detected for a difference in the expression by the method as defined in any one of claims 2 to 4.

7. A kit for use in the method as defined in claim 1, comprising an adaptor comprising an oligodeoxyribonucleotide to be attached to both ends of DNA fragments in a sample, a restriction enzyme having a cleavage site in the adaptor, and a primer for PCR having a nucleotide sequence complementary to a nucleotide sequence of the adaptor before cleavage with the restriction enzyme.


SEQUENCE LISTING

<110> Eisai Co., Ltd.

<120> Method of normalizing DNA fragment contents and subtraction method

<130> OP1333-PCT

<150> JP 2001-184757
<151> 2001-06-19

<160> 12

<210> 1
<211> 26
<212> DNA
<213> Artificial Sequence

<220>
<223> synthetic DNA

<400> 1
cagctccaca acctacatca ttccgt                                                26

<210> 2
<211> 12
<212> DNA
<213> Artificial Sequence

<220>
<223> synthetic DNA

<400> 2
acggaatgat gt                                                               12

<210> 3
<211> 26

<212> DNA
<213> Artificial Sequence

<220>
<223> synthetic DNA

<400> 3
gtccatcttc tctctgagac tctggt                                          26


<210> 4
<211> 12
<212> DNA
<213> Artificial Sequence

<220>
<223> synthetic DNA

<400> 4
accagagtct ca                                                         12


<210> 5
<211> 26
<212> DNA
<213> Artificial Sequence

<220>
<223> synthetic DNA

<400> 5
ctgatgggtg tcttctgtga gtgtgt                                          26


<210> 6
<211> 12
<212> DNA
<213> Artificial Sequence

<220>

<223> synthetic DNA

<400> 6
acacactcac ag                                                    12

<210> 7
<211> 26
<212> DNA
<213> Artificial Sequence

<220>
<223> synthetic DNA

<400> 7
ccagcatcga gaatcagtgt gacagt                                     26

<210> 8
<211> 12
<212> DNA
<213> Artificial Sequence

<220>
<223> synthetic DNA

<400> 8
actgtcacac tg                                                    12

<210> 9
<211> 26
<212> DNA
<213> Artificial Sequence

<220>
<223> synthetic DNA

<400> 9
gtcgatgaac ttcgactgtc gatcgt                                     26

<210> 10
<211> 12
<212> DNA
<213> Artificial Sequence

<220>
<223> synthetic DNA

<400> 10
acgatcgaca gt                                                          12


<210> 11
<211> 26
<212> DNA
<213> Artificial Sequence

<220>
<223> synthetic DNA

<400> 11
agtgctacgt acacatgtct ggctgt                                           26


<210> 12
<211>
<212> DNA
<213> Artificial Sequence

<220>
<223> synthetic DNA

<400> 12
acagccagac at                                                          12